# EUROPEAN PATENT APPLICATION

(11) **EP 4 134 726 A1**
(43) Date of publication of application: **15.02.2023**
(21) Application number: 21872035.7
(22) Date of filing: 18.08.2021
(51) Int. Cl.: G02B 23/24, A61B 1/005, A61B 1/008

(54) **ENDOSCOPE**

(30) Priority: 25.09.2020 JP 2020160963
(71) Applicant: HOYA CORPORATION, Shinjuku-ku Tokyo 160-8347 (JP)
(72) Inventor: OCHI, Kunitaka, Tokyo 160-8347 (JP)
(74) Representative: Lucke, Andreas
(86) International application number: PCT/JP2021/030248
(87) International publication number: WO 2022/064903

(57) **Abstract**

An occurrence of a defect in being bent at a connection portion between an active bending section and a passive bending section is prevented beforehand.

In an endoscope including: an active bending section and a passive bending section that are bendable and sequentially disposed from a distal tip; and a connection cylinder (50) for connecting the active bending section and the passive bending section, the active bending section and the passive bending section each include a plurality of articulated cylinders (30, 40), a coil member (60), through which an operation wire (90) to be operated for bending the active bending section is inserted, is fixed to the connection cylinder (50), and a cutout (43) is formed in a vicinity of the coil member (60), the cutout (43) enhancing a rotational freedom degree of an articulated cylinder (40A), in the passive bending section, to be connected with the connection cylinder (50) and to rotate.

## Description

### Technical Field

The present invention relates to an endoscope including a first bending section and a second bending section that are bendable.

The present application claims priority based on Japanese Patent Application No. 2020-160963 filed on September 25, 2020, the entire contents of which are incorporated herein by reference.

### Background Art

Conventionally, an endoscope, in which an insertion portion to be inserted into a body lumen includes an active bending section (first bending section) and a passive bending section (second bending section) sequentially from a distal tip side, is widely used.

For example, Patent Literature 1 discloses an endoscope configured such that a curvature value at the time of being bent decreases from the active bending section toward the passive bending section, so that the insertion portion has a gentle curvature change.

In addition, Patent Literature 2 discloses an endoscope that can be operated by a user operating an operation unit so that the active bending section and the passive bending section are each bendable in four directions.

### Citation List

### Patent Literature

Patent Literature 1: JP 2006-218231 A
Patent Literature 2: JP 2013-202304 A

### Summary of Invention

### Technical Problem

The active bending section and the passive bending section each include a plurality of articulated cylinders, and are connected through a connection cylinder. In addition, a coil member, into which a wire to be operated for bending the active bending section is inserted, is fixed to the connection cylinder by welding, for example. Since the coil member is excellent in deformation property and recovery property, the coil member does not hinder bending or recovery at the connection portion between the active bending section and the passive bending section.

On the other hand, as described above, in a case where the coil member is welded and fixed, the deformation of the coil member is limited in the welded part. Hence, in a case where the articulated cylinder of the active bending section or the passive bending section interferes with the coil member, bending and recovery at the connection portion of the active bending section or the passive bending section may be hindered.
However, in the endoscopes of Patent Literature 1 and Patent Literature 2, such a drawback is not devised, and cannot be solved.

The present invention has been made in view of such circumstances, and has an object to provide an endoscope capable of preventing beforehand an occurrence of a defect in being bent at a connection portion between an active bending section and a passive bending section, even in a case where a coil member is fixed, and deformation of the coil member is limited.

### Solution to Problem

An endoscope according to the present invention including: a first bending section and a second bending section that are bendable and sequentially disposed from a distal tip; and a connection cylinder for connecting the first bending section and the second bending section, in which the first bending section and the second bending section each include a plurality of articulated cylinders, a coil member, through which a wire to be operated for bending the first bending section is inserted, is fixed to the connection cylinder, and a cutout is formed in a vicinity of the coil member, the cutout enhancing a rotational freedom degree of a predetermined articulated cylinder, in the second bending section, to be connected with the connection cylinder and to rotate.

In the present invention, the cutout is formed in the vicinity of the coil member in the predetermined articulated cylinder or the connection cylinder. With such a cutout, interference between the coil member and the predetermined articulated cylinder can be eliminated or hindrance by the coil member to the rotation of the predetermined articulated cylinder can be suppressed, and rotational freedom degree of the predetermined articulated cylinder is enhanced.

### Advantageous Effects of Invention

According to the present invention, even in a case where the coil member is fixed and deformation of the coil member is limited, an occurrence of a defect in being bent at the connection portion between the active bending section and the passive bending section can be prevented beforehand.

### Brief Description of Drawings

Fig. 1 is an external view of an endoscope according to a first embodiment of the present invention.
Fig. 2 is a cross-sectional view of an active bending section and a passive bending section along an axial direction of an insertion portion.
Fig. 3 is a cross-sectional view illustrating a connection state of an articulated cylinder, a connection cylinder, and an articulated cylinder.
Fig. 4 is a perspective view illustrating an outer appearance of the articulated cylinder.
Fig. 5 is a cross-sectional view illustrating a connection state of an articulated cylinder, a connection cylinder, and an articulated cylinder of an endoscope according to a second embodiment.
Fig. 6 is a cross-sectional view illustrating a connection state of an articulated cylinder, a connection cylinder, and an articulated cylinder of an endoscope according to a third embodiment.
Fig. 7 is a cross-sectional view illustrating a connection state of an articulated cylinder, a connection cylinder, and an articulated cylinder of an endoscope according to a fourth embodiment.
Fig. 8 is a cross-sectional view taken along line VIII-VIII in Fig. 7.
Fig. 9 is a cross-sectional view illustrating a connection state of an articulated cylinder, a connection cylinder, and an articulated cylinder of an endoscope according to a fifth embodiment.

### Description of Embodiments

Hereinafter, an endoscope according to embodiments of the invention will be described in detail with reference to the drawings.

### (First Embodiment)

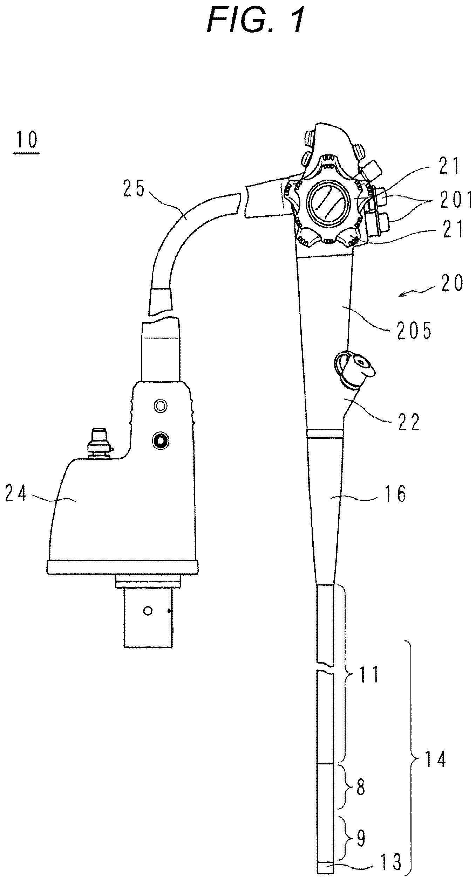

Fig. 1 is an external view of an endoscope 10 according to a first embodiment of the present invention. The endoscope 10 according to the present embodiment includes an imaging means. The endoscope 10 includes an insertion portion 14 to be inserted into a body lumen of a subject, an operation unit 20 for operating the insertion portion 14, and a connector unit 24 connected with a processor, a light source device, an air/water supply device, and the like, which are not illustrated.

The insertion portion 14 is connected with the operation unit 20 through a bend preventing portion 16, and the operation unit 20 is connected with the connector unit 24 through a universal cord 25.

The universal cord 25 has pliability, and includes an electric line that sends an electric signal from the imaging means of the insertion portion 14 to the connector unit 24, and a water passage through which water supplied from the connector unit 24 passes and an air passage through which air passes.

The operation unit 20 includes a grip portion 205, buttons 201 each for receiving an instruction such as water supply or air supply from the user, and a bending knob 21 for operating bending of an active bending section 9 to be described later.

The grip portion 205 has a substantially cylindrical shape, and is reduced in diameter toward the insertion portion 14. The grip portion 205 is provided with a channel inlet 22, into which a treatment tool or the like is to be inserted, near the insertion portion 14 side.

The insertion portion 14 has a cylindrical shape with a small diameter, and is configured to be bendable. The insertion portion 14 includes a distal tip 13, the active bending section 9 (first bending section), a passive bending section 8 (second bending section), and a flexible portion 11 sequentially in this order from the distal tip side.

The distal tip 13 includes an imaging unit (not illustrated) including an imaging means such as a charge coupled device (CCD) or a complementary metal oxide semiconductor (CMOS), a circuit board for driving the imaging means, an observation optical system, and the like. In addition, the distal tip 13 includes an illumination unit (not illustrated) including an irradiation and/or illumination optical system or the like for irradiating an observation target site in the body lumen.

The active bending section 9 is actively bendable. That is to say, the active bending section 9 is bent in four directions in accordance with the operation of the bending knob 21. On the other hand, the passive bending section 8 is passively bent. That is to say, the passive bending section 8 is bent in any of the four directions by contact with the subject. The flexible portion 11 has flexibility, and is flexible.

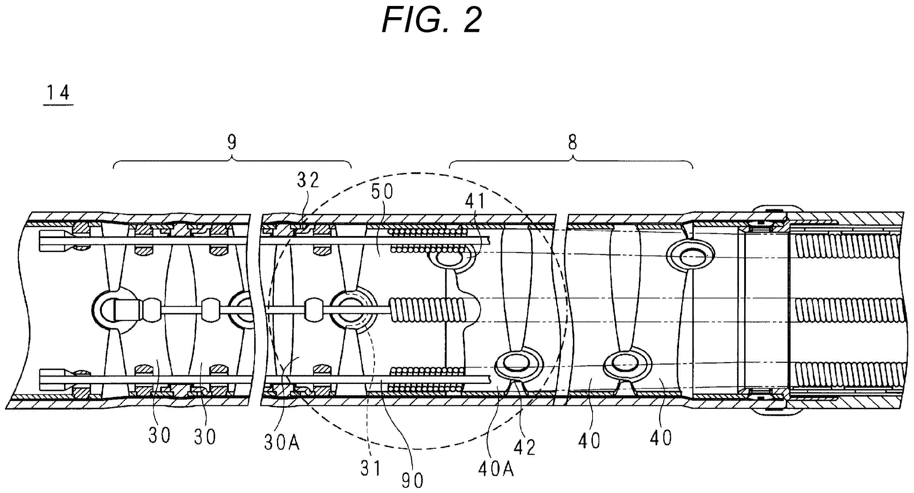

Fig. 2 is a cross-sectional view of the active bending section 9 and the passive bending section 8 along an axial direction of the insertion portion 14. In particular, Fig. 2 illustrates a boundary part between the active bending section 9 and the passive bending section 8.

The active bending section 9 has a plurality of articulated cylinders 30. The plurality of articulated cylinders 30 are connected in alignment. Each articulated cylinder 30 has a substantially cylindrical shape, and is pivotally attached to an adjacent articulated cylinder 30 so as to be rotatable vertically and horizontally.

In addition, each articulated cylinder 30 is provided with a pair of bearings 31 for receiving one rotary shaft on one end side in the axial direction, and a pair of bearings 32 for receiving another rotary shaft, orthogonal to the afore-mentioned one rotary shaft, on the other end side in the axial direction. The pair of bearings 31 and the pair of bearings 32 each have a substantially disk shape with a through hole in a central part, and extend in the axial direction of the articulated cylinders 30.
The plurality of articulated cylinders 30 are connected in the alignment direction such that the pair of bearings 31 and the pair of bearings 32 are alternately positioned.

The passive bending section 8 includes a plurality of articulated cylinders 40. The plurality of articulated cylinders 40 are connected in alignment. Each articulated cylinder 40 has a substantially cylindrical shape, and is pivotally attached to an adjacent articulated cylinder 40 so as to be rotatable vertically and horizontally.

In addition, each articulated cylinder 40 is provided with a pair of bearings 41 for receiving one rotary shaft on one end side in the axial direction, and a pair of bearings 42 for receiving another rotary shaft, orthogonal to the afore-mentioned one rotary shaft, on the other end side in the axial direction. The pair of bearings 41 and the pair of bearings 42 each have a substantially disk shape with a through hole in a central portion, are displaced in the circumferential direction with respect to the pair of bearings 31 and the pair of bearings 32, and extend in the axial direction of the articulated cylinder 40. The plurality of articulated cylinders 40 are connected in the alignment direction such that the pair of bearings 41 and the pair of bearings 42 are alternately positioned.

Four operation wires 90, which are operated to bend the active bending section 9, are inserted inside the flexible portion 11, the active bending section 9, and the passive bending section 8. One end of each operation wire 90 is fixed to an end portion on the distal tip 13 side of the active bending section 9, and the other end is connected with the bending knob 21 of the operation unit 20. The outside of the plurality of articulated cylinders 30 and the outside of the plurality of articulated cylinders 40 are covered with bending rubber.

A connection cylinder 50 is interposed between the active bending section 9 and the passive bending section 8. That is to say, the articulated cylinder 30 (hereinafter, referred to as an articulated cylinder 30A) closest to the passive bending section 8 side among the plurality of articulated cylinders 30 in the active bending section 9 and the articulated cylinder 40 (hereinafter, referred to as an articulated cylinder 40A) closest to the active bending section 9 side among the plurality of articulated cylinders 40 of the passive bending section 8 are connected with each other through the connection cylinder 50.

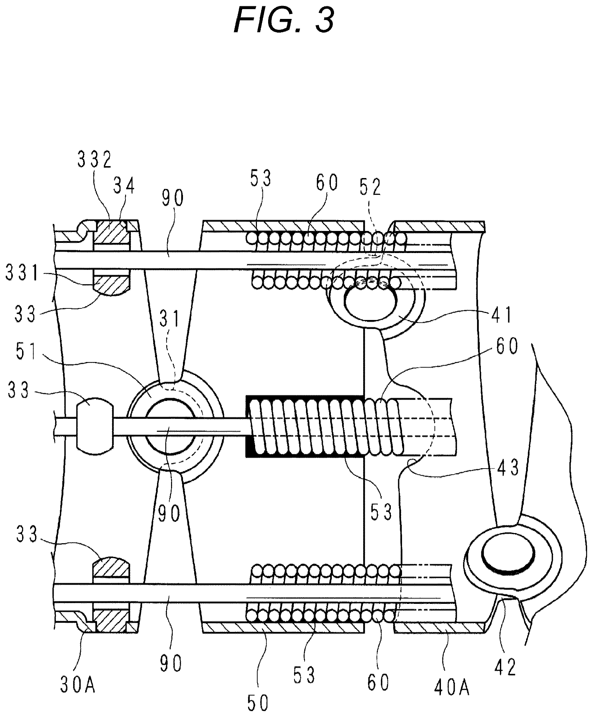

Fig. 3 is a cross-sectional view illustrating a connection state of the articulated cylinder 30A, the connection cylinder 50, and the articulated cylinder 40A (a predetermined articulated cylinder). Fig. 3 illustrates a part surrounded by a broken line circle in Fig. 2, in an enlarged manner.

On an inner circumferential surface of the articulated cylinder 30A, wire guides 33 for respectively holding the operation wires 90 are provided at four positions. In the articulated cylinder 30A, in order to fix the wire guides 33, through holes 34, which penetrate through the articulated cylinder 30A between the inside and the outside, are provided at four positions. The through holes 34 are provided at equal intervals in the circumferential direction of the articulated cylinder 30A. Each wire guide 33 is engaged with the through hole 34.

The wire guide 33 includes a holding portion 331 having a ring shape, and a fitted portion 332 having a cylindrical shape and extending outward in the radial direction of the holding portion 331. The fitted portion 332 is fitted into the through hole 34, and the operation wire 90 is inserted into the holding portion 331.
Among the four wire guides 33, two wire guides 33 facing each other are provided in the vicinity of the pair of bearings 31.

The connection cylinder 50 has a substantially cylindrical shape, and a pair of bearings 51, which are pivotally attached to the pair of bearings 31 of the articulated cylinder 30A, extend in the axial direction at one end on the articulated cylinder 30A side. The pair of bearings 51 each have a substantially disk shape with a through hole in a central part. Regarding the connection cylinder 50, as separating from the pair of bearings 51 in the circumferential direction, the axial dimension is smaller.

In addition, on the inner circumferential surface of the connection cylinder 50, coil members 60 for guiding the operation wires 90 are provided at four positions. The coil members 60 are provided at equal intervals in the circumferential direction of the connection cylinder 50. For example, the coil members 60 are fixed to the inner circumferential surface of the connection cylinder 50 by welding. That is to say, a welded portion 53 is interposed between the coil member 60 and the connection cylinder 50.

Each coil member 60 has a pipe shape with a coil wire tightly wound around. Each coil member 60 extends along the axial direction of the connection cylinder 50, and an end portion of the coil member 60 protrudes from the other end of the connection cylinder 50 and reaches the inside of the articulated cylinder 40A. The operation wire 90 is inserted through the coil member 60.

Furthermore, in the connection cylinder 50, a pair of bearings 52, which are pivotally attached to the pair of bearings 41 of the articulated cylinder 40A, extend in the axial direction at the other end on the articulated cylinder 40A side. The pair of bearings 52 each have a substantially disk shape with a through hole in a central part. The pair of bearings 52 are provided between the coil members 60, which are adjacent to each other.

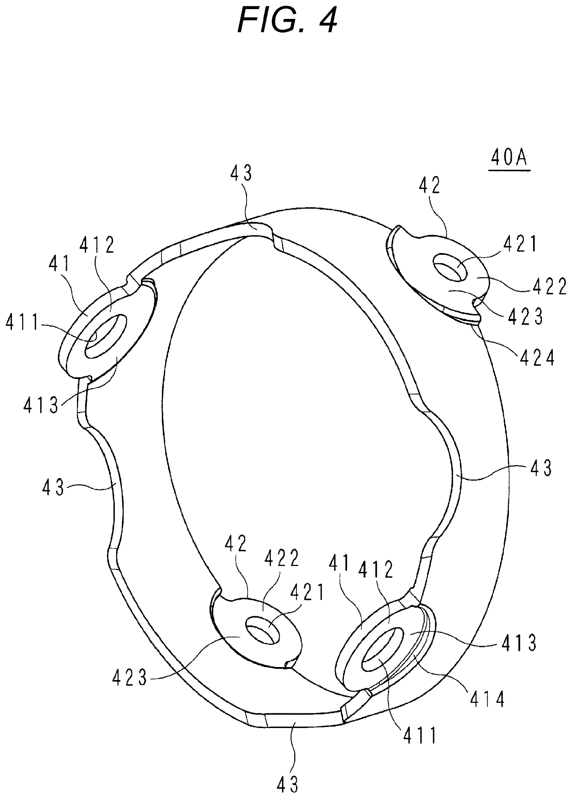

Fig. 4 is a perspective view illustrating an outer appearance of the articulated cylinder 40A.
The articulated cylinder 40A has a substantially cylindrical shape, and the pair of bearings 41 are provided at one end on the connection cylinder 50 side, and are pivotally attached to the pair of bearings 52 of the connection cylinder 50. Therefore, the articulated cylinder 40A rotates around the axis passing through the pair of bearings 41.

The bearing 41 is flat, and includes a protruding portion 412 having a semicircular shape, and a base portion 413 having a semicircular shape with the diameter larger than that of the protruding portion 412, and a through hole 411 is formed in a central part. The bearing 41 is disposed slightly on an axial center side than the outer circumferential surface of the articulated cylinder 40A, and a guide step 414 is formed between the base portion 413 and the outer circumferential surface of the articulated cylinder 40A. The height of the guide step 414 decreases, as approaching the afore-mentioned one end of the articulated cylinder 40A.

As described above, since the bearing 41 is disposed slightly on the axial center side than the outer circumferential surface of the articulated cylinder 40A, the base portion 413 slightly protrudes on the axial center side as compared with the inner circumferential surface of the articulated cylinder 40A. Accordingly, a step (not illustrated) similar to the guide step 414 is also formed between the base portion 413 and the inner circumferential surface of the articulated cylinder 40A.

Further, at the afore-mentioned one end of the articulated cylinder 40A, four cutouts 43 (first cutout), which enhance the rotational freedom degree of the articulated cylinder 40A, are formed. Each cutout 43 has a semicircular shape with a diameter larger than the diameter of the coil member 60, for example.

Two cutouts 43 are formed to be spaced apart from each other between the pair of bearings 41. When the articulated cylinder 40A is connected with the connection cylinder 50, each cutout 43 is configured to be disposed in the vicinity of the coil member 60 of the connection cylinder 50. In detail, the cutout 43 is formed at a position corresponding to the coil member 60 (the welded portion 53) in the circumferential direction of the articulated cylinder 40A.

Furthermore, as described above, the pair of bearings 42 are provided at the other end of the articulated cylinder 40A. The bearing 42 is flat, and includes a protruding portion 422 having a semicircular shape, and a base portion 423 having a semicircular shape with the diameter larger than that of the protruding portion 422, and a through hole 421 is formed in a central part. The bearing 42 is disposed slightly on an outer side in the radial direction than the outer circumferential surface of the articulated cylinder 40A, and a step 424 is formed between the base portion 423 and the outer circumferential surface of the articulated cylinder 40A. The height of the step 424 increases, as approaching the other end of the articulated cylinder 40A.

In this manner, since the bearing 42 is disposed slightly on the outer side in the radial direction than the outer circumferential surface of the articulated cylinder 40A, the base portion 423 is disposed slightly on the outer side than the inner circumferential surface of the articulated cylinder 40A. Accordingly, a step (not illustrated) similar to the guide step 424 is also formed between the base portion 423 and the inner circumferential surface of the articulated cylinder 40A.

When the articulated cylinder 40A is connected with the connection cylinder 50, the bearing 52 of the connection cylinder 50 is guided by the guide step 414 of the articulated cylinder 40A, and is disposed coaxially with the bearing 41 and pivotally attached to be rotatable. Further, when the articulated cylinder 40A is connected with an adjacent articulated cylinder 40, the bearing 42 of the articulated cylinder 40A is pivotally attached to the bearing of the adjacent articulated cylinder 40 to be rotatable.

As described above, the coil member 60 is fixed to the inner circumferential surface of the connection cylinder 50 by welding. Therefore, in the coil member 60, the welded part is not deformable, and a freely deformable part is limited to the end portion on the articulated cylinder 40A side. The deformation freedom degree of the coil member 60 is greatly inferior. Hence, the rotation of the articulated cylinder 40A, which is pivotally attached to the connection cylinder 50 and rotates, may be hindered by interference with the coil member 60 (the welded part).

In contrast, in the endoscope 10 in the first embodiment, as described above, in the articulated cylinder 40A, the cutout 43 is formed at a position corresponding to the coil member 60 in the circumferential direction of the articulated cylinder 40A, in the vicinity of the coil member 60 of the connection cylinder 50. Therefore, when the articulated cylinder 40A rotates, it is possible to prevent the articulated cylinder 40A from interfering with the coil member 60 with certainty.
Therefore, in the endoscope 10 in the first embodiment, while the operation wire 90 is being guided with certainty by use of the coil member 60 having a long natural length, an occurrence of a defect in being bent and recovered at the connection portion between the active bending section 9 and the passive bending section 8 can be prevented beforehand.

Heretofore, a case where the cutout 43 has a semicircular shape has been described as an example, but the present invention is not limited to this, and may have a rectangular shape, for example.

Further, heretofore, a case where four cutouts 43 are formed at the afore-mentioned one end of the articulated cylinder 40A has been described as an example, but the present invention is not limited to this. The number of the cutouts 43 may be five or more, or may be three or less.
Furthermore, heretofore, a case where all of four cutouts 43 have the same shape has been described as an example, but the present invention is not limited to this, and the shapes and sizes may be configured to be different from one another.

In addition, heretofore, in a case where the coil member 60 is formed in the connection portion between the active bending section 9 and the passive bending section 8, the cutout 43 provided to prevent interference with the coil member 60 has been described, but the present invention is not limited to this. For example, it is needless to say that the present invention is also applicable to a case where the coil member 60 is formed at a connection portion between the passive bending section 8 and the flexible portion 11.

### (Second Embodiment)

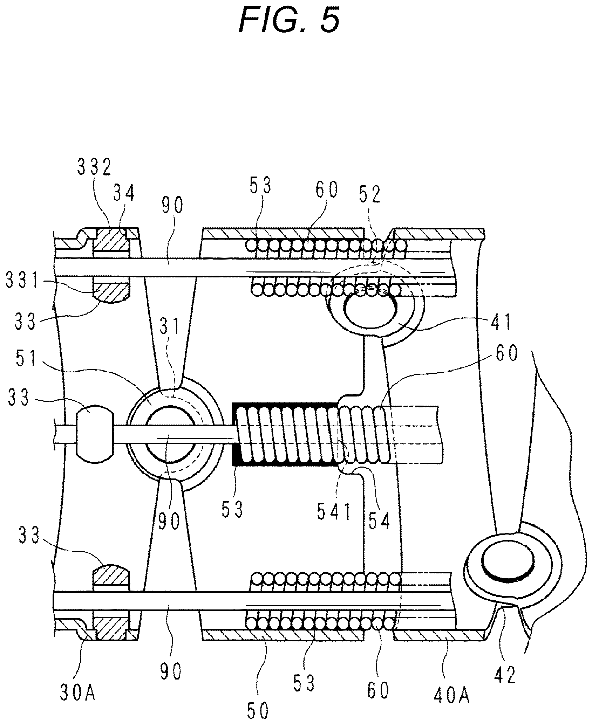

Fig. 5 is a cross-sectional view illustrating a connection state of the articulated cylinder 30A, the connection cylinder 50, and the articulated cylinder 40A of the endoscope 10 according to a second embodiment. Similarly to the endoscope 10 in the first embodiment, the articulated cylinder 40A is pivotally attached to the connection cylinder 50, and the articulated cylinder 30A is also pivotally attached to the connection cylinder 50. In addition, the coil member 60 is welded to the inner circumferential surface of the connection cylinder 50, and the coil member 60 extends to the inside of the articulated cylinder 40A.

In the endoscope 10 according to the second embodiment, in the connection cylinder 50, four cutouts 54 (second cutout), which enhance the rotational freedom degree of the articulated cylinder 40A, are formed at the other end on the articulated cylinder 40A side. Each cutout 54 has, for example, a chamfered rectangular shape, and the dimension in the circumferential direction of the connection cylinder 50 is larger than the diameter of the coil member 60. The cutouts 54 are formed at equal intervals in the circumferential direction of the connection cylinder 50.

Each cutout 54 is disposed in the vicinity of the coil member 60. In detail, the cutout 54 is formed at a position corresponding to the coil member 60 in the circumferential direction of the connection cylinder 50 from the other end on the articulated cylinder 40A side in the axial direction of the connection cylinder 50.

As described above, since the coil member 60 is fixed to the inner circumferential surface of the connection cylinder 50 by welding, the welded part of the coil member 60 is not deformable. Therefore, the rotation of the articulated cylinder 40A may be hindered by interference with the coil member 60.

In contrast, in the endoscope 10 in the second embodiment, as described above, in the connection cylinder 50, the cutout 54 is formed at a position corresponding to the coil member 60 in the circumferential direction of the connection cylinder 50. Therefore, in the coil member 60, a part where deformation is limited by welding is reduced. In addition, it is possible to ensure a wide interval from an edge 541 on the articulated cylinder 30A side of the cutout 54 to the articulated cylinder 40A. Therefore, at the time of rotating, the articulated cylinder 40A is capable of rotating without interfering with the welded part of the coil member 60 and without being hindered by the coil member 60.
Therefore, in the endoscope 10 in the second embodiment, while the operation wire 90 is being guided with certainty by use of the coil member 60 having a long natural length, an occurrence of a defect in being bent and recovered at the connection portion between the active bending section 9 and the passive bending section 8 can be prevented beforehand.

Heretofore, a case where the cutout 54 has a rectangular shape has been described as an example, but the present invention is not limited to this, and may have a semicircular shape, for example.

The same portions as those in the first embodiment are denoted by the same reference numerals, and detailed descriptions thereof will be omitted.

### (Third Embodiment)

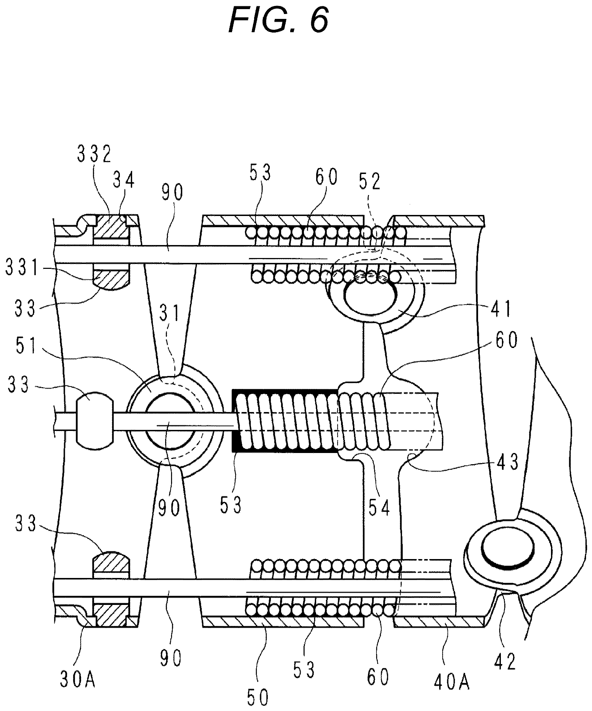

Fig. 6 is a cross-sectional view illustrating a connection state of the articulated cylinder 30A, the connection cylinder 50, and the articulated cylinder 40A of the endoscope 10 according to a third embodiment. Similarly to the endoscope 10 in the first embodiment, the articulated cylinder 40A is pivotally attached to the connection cylinder 50, and the articulated cylinder 30A is also pivotally attached to the connection cylinder 50. In addition, the coil member 60 is welded to the inner circumferential surface of the connection cylinder 50, and the coil member 60 extends to the inside of the articulated cylinder 40A.

In the endoscope 10 according to the third embodiment, in the connection cylinder 50, four cutouts 54 (second cutout), which enhance the rotational freedom degree of the articulated cylinder 40A, are formed at the other end on the articulated cylinder 40A side. Each cutout 54 has, for example, a rectangular shape. The cutout 54 has already been described in the second embodiment, and a detailed description thereof will be omitted.

In addition, four cutouts 43 (first cutout), which enhance the rotational freedom degree of the articulated cylinder 40A, are formed at one end on the connection cylinder 50 side of the articulated cylinder 40A. Each cutout 43 has, for example, a semicircular shape. The cutout 43 has already been described in the first embodiment, and a detailed description thereof will be omitted.

As described above, since the coil member 60 is fixed to the inner circumferential surface of the connection cylinder 50 by welding, the welded part of the coil member 60 is not deformable. Therefore, the rotation of the articulated cylinder 40A may be hindered by interference with the welded part of the coil member 60.

In contrast, in the endoscope 10 in the third embodiment, as described above, in the connection cylinder 50, the cutout 54 is formed at a position corresponding to the coil member 60 in the circumferential direction of the connection cylinder 50. In addition, in the articulated cylinder 40A, the cutout 43 is formed at a position corresponding to the coil member 60 in the circumferential direction.

Therefore, the articulated cylinder 40A is capable of rotating without interfering with the welded part of the coil member 60 and without being hindered by the coil member 60. Therefore, in the endoscope 10 in the third embodiment, while the operation wire 90 is being guided with certainty by use of the coil member 60 having a long natural length, an occurrence of a defect in being bent and recovered at the connection portion between the active bending section 9 and the passive bending section 8 can be prevented beforehand.

The same portions as those in the first embodiment are denoted by the same reference numerals, and detailed descriptions thereof will be omitted.

### (Fourth Embodiment)

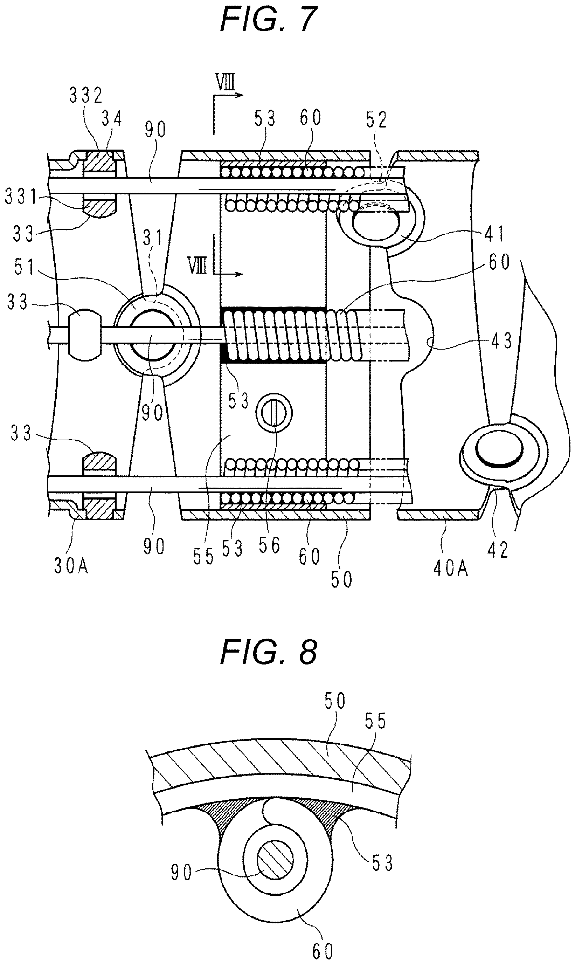

Fig. 7 is a cross-sectional view illustrating a connection state of the articulated cylinder 30A, the connection cylinder 50, and the articulated cylinder 40A of the endoscope 10 according to a fourth embodiment. Fig. 8 is a cross-sectional view taken along line VIII-VIII in Fig. 7. Similarly to the endoscope 10 in the first embodiment, the articulated cylinder 40A is pivotally attached to the connection cylinder 50, and the articulated cylinder 30A is also pivotally attached to the connection cylinder 50.

The endoscope 10 in the fourth embodiment includes an anchoring circular ring 55 for fixing the coil member 60. The anchoring circular ring 55 has a cylindrical shape, and the axial dimension of the anchoring circular ring 55 is smaller than the axial dimension of the connection cylinder 50. In addition, the outer diameter of the anchoring circular ring 55 is slightly smaller than the inner diameter of the connection cylinder 50, and is fitted in the connection cylinder 50. For example, the anchoring circular ring 55 is screwed to the connection cylinder 50 by a screw 56.

In the coil member 60, the coil members 60 are provided at four positions on the inner circumferential surface of the anchoring circular ring 55. The coil members 60 are provided at equal intervals in the circumferential direction of the anchoring circular ring 55. For example, the coil member 60 is welded to the inner circumferential surface of the anchoring circular ring 55. That is to say, as illustrated in Fig. 8, the welded portion 53 is interposed between the coil member 60 and the anchoring circular ring 55, and the outer circumferential surface of the anchoring circular ring 55 is covered with the connection cylinder 50. The operation wire 90 is inserted through the coil member 60.

In the endoscope 10 according to the fourth embodiment, four cutouts 43 (first cutout) that enhance the rotational freedom degree of the articulated cylinder 40A are formed at one end of the articulated cylinder 40A on the connection cylinder 50 side. Each cutout 43 has, for example, a semicircular shape. The cutout 43 has already been described in the first embodiment, and a detailed description thereof will be omitted.

As described above, since the coil member 60 is fixed to the inner circumferential surface of the anchoring circular ring 55 by welding, the welded part of the coil member 60 is not deformable. Therefore, the rotation of the articulated cylinder 40A may be hindered by interference with the welded part of the coil member 60.

In contrast, in the endoscope 10 in the fourth embodiment, in the articulated cylinder 40A, the cutout 43 is formed at a position corresponding to the coil member 60 in the circumferential direction. Therefore, the articulated cylinder 40A is capable of rotating without interfering with the welded part of the coil member 60 and without being hindered by the coil member 60. Therefore, in the endoscope 10 in the fourth embodiment, while the operation wire 90 is being guided with certainty by use of the coil member 60 having a long natural length, an occurrence of a defect in being bent and recovered at the connection portion between the active bending section 9 and the passive bending section 8 can be prevented beforehand.

The same portions as those in the first embodiment are denoted by the same reference numerals, and detailed descriptions thereof will be omitted.

### (Fifth Embodiment)

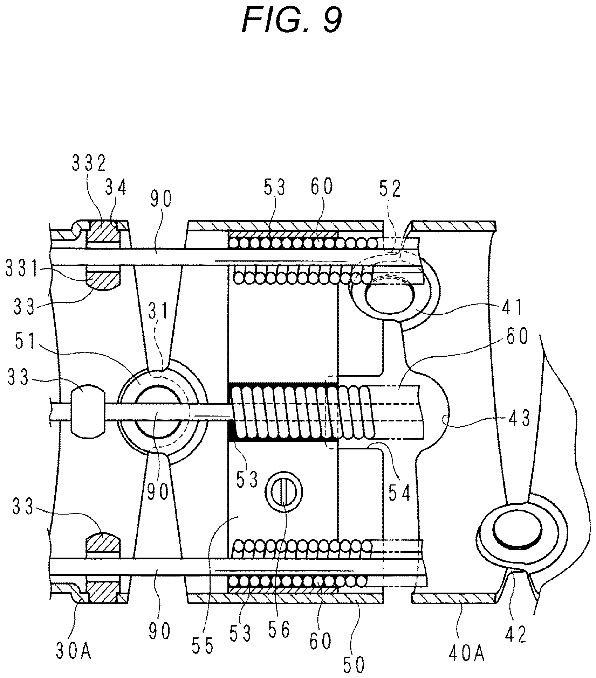

Fig. 9 is a cross-sectional view illustrating a connection state of the articulated cylinder 30A, the connection cylinder 50, and the articulated cylinder 40A of the endoscope 10 according to a fifth embodiment. Similarly to the endoscope 10 in the first embodiment, the articulated cylinder 40A is pivotally attached to the connection cylinder 50, and the articulated cylinder 30A is also pivotally attached to the connection cylinder 50. In addition, similarly to the fourth embodiment, the anchoring circular ring 55 is provided, and the coil member 60 is welded to the inner circumferential surface of the anchoring circular ring 55.

In the endoscope 10 according to the fifth embodiment, in the connection cylinder 50, four cutouts 54 (second cutout), which enhance the rotational freedom degree of the articulated cylinder 40A, are formed at the other end on the articulated cylinder 40A side. Each cutout 54 has, for example, a rectangular shape. The cutout 54 has already been described in the second embodiment, and a detailed description thereof will be omitted.

In addition, four cutouts 43 (first cutout), which enhance the rotational freedom degree of the articulated cylinder 40A, are formed at one end on the connection cylinder 50 side of the articulated cylinder 40A. Each cutout 43 has, for example, a semicircular shape. The cutout 43 has already been described in the first embodiment, and a detailed description thereof will be omitted.

As described above, since the coil member 60 is welded to the inner circumferential surface of the anchoring circular ring 55, the welded part of the coil member 60 is not deformable. Therefore, the rotation of the articulated cylinder 40A may be hindered by interference with the welded part of the coil member 60.

In contrast, in the endoscope 10 in the fifth embodiment, the cutout 54 is formed in the connection cylinder 50, and the cutout 43 is formed in the articulated cylinder 40A. Therefore, the articulated cylinder 40A is capable of rotating without interfering with the welded part of the coil member 60 and without being hindered by the coil member 60. Therefore, in the endoscope 10 in the fifth embodiment, while the operation wire 90 is being guided with certainty by use of the coil member 60 having a long natural length, an occurrence of a defect in being bent and recovered at the connection portion between the active bending section 9 and the passive bending section 8 can be prevented beforehand.

The same portions as those in the first embodiment are denoted by the same reference numerals, and detailed descriptions thereof will be omitted.

Technical features (constitutional requirements) that have been described in the first to fifth embodiments can be combined with one another, and a new technical feature can be formed with the combination.
The embodiments that have been disclosed herein should be considered to be exemplary in all respects without being limited. The scope of the present invention is indicated by the scope of claims, not the above-mentioned meaning, and is intended to include all modifications within the meaning and scope equivalent to the claims.

### Reference Signs List

- 8: Passive bending section
- 9: Active bending section
- 10: Endoscope
- 13: Distal tip
- 14: Insertion portion
- 30, 30A, 40, 40A: Articulated cylinder
- 43: Cutout
- 50: Connection cylinder
- 54: Cutout
- 60: Coil member
- 90: Operation wire

## Claims

1. An endoscope comprising: a first bending section and a second bending section that are bendable and sequentially disposed from a distal tip; and a connection cylinder for connecting the first bending section and the second bending section, wherein
the first bending section and the second bending section each include a plurality of articulated cylinders,
a coil member, through which a wire to be operated for bending the first bending section is inserted, is fixed to the connection cylinder, and
a cutout is formed in a vicinity of the coil member, the cutout enhancing a rotational freedom degree of a predetermined articulated cylinder, in the second bending section, to be connected with the connection cylinder and to rotate.

2. The endoscope according to claim 1, wherein
the coil member is fixed to an inner circumferential surface of the connection cylinder, and extends in an axial direction of the connection cylinder, and
the cutout includes
a first cutout formed at a position corresponding to the coil member at an edge on a connection cylinder side of the predetermined articulated cylinder.

3. The endoscope according to claim 1, wherein
the coil member is fixed to an inner circumferential surface of the connection cylinder, and extends in an axial direction of the connection cylinder, and
the cutout includes
a second cutout formed at a position corresponding to the coil member at an edge on a predetermined articulated cylinder side of the connection cylinder.

4. The endoscope according to claim 2, wherein
the cutout includes
a second cutout formed at a position corresponding to the coil member at an edge on a predetermined articulated cylinder side of the connection cylinder.

5. The endoscope according to claim 2 or 4, wherein the first cutout has a semicircular shape with a diameter larger than a diameter of the coil member.

6. The endoscope according to claim 3 or 4, wherein the second cutout has a chamfered rectangular shape with a dimension in a circumferential direction of the connection cylinder that is longer than a diameter of the coil member.
